Europäisches Patentamt

(19) European Patent Office

Office européen des brevets

(11) **EP 0 922 757 A1**

(12) **EUROPEAN PATENT APPLICATION**
published in accordance with Art. 158(3) EPC

(43) Date of publication:
16.06.1999 Bulletin 1999/24

(51) Int. Cl.$^6$: **C12N 1/16**
// C07C31/24

(21) Application number: 98911185.1

(22) Date of filing: 02.04.1998

(86) International application number:
PCT/JP98/01534

(87) International publication number:
WO 98/44089 (08.10.1998 Gazette 1998/40)

(84) Designated Contracting States:
**DE FR GB**

(30) Priority: 02.04.1997 JP 8372697

(71) Applicant:
**MITSUBISHI CHEMICAL CORPORATION**
**Chiyoda-ku, Tokyo 100-0005 (JP)**

(72) Inventors:
• **CHO, Hiroshi-Mitsubishi Chemical Corporation**
**Yokohama-shi,Kanagawa 227-0033 (JP)**

• **YAMAGISHI, Kenji**
**Mitsubishi Chemical Corporation**
**Yahatanishi-ku,Kitakyushu-shi Fukuoka (JP)**
• **MIKAWA, Takashi-Mitsubishi Chemical**
**Corporation**
**Yokohama-shi, Kanagawa 227-0033 (JP)**

(74) Representative:
**VOSSIUS & PARTNER**
**Postfach 86 07 67**
**81634 München (DE)**

(54) **ERYTHRITOL-PRODUCING MICROORGANISM AND PROCESS FOR PRODUCING THE SAME**

(57) A microorganism which does not form substantial foams during aerobic cultivation and which has an ability of producing erythritol is obtained by cultivating a microorganism having an ability of producing erythritol in a liquid medium, removing a microbial aggregate from the culture, collecting a microorganism which has physical properties such that when fractionated with water and a water-insoluble solvent the microorganism remains in a water layer in an amount of at least 20% or which has a hydrophobicity of 80% or less and further collecting a microorganism which does not form substantial foams during aerobic cultivation.

EP 0 922 757 A1

**Description**

Technical Field

The present invention relates to a method of producing microorganism which produces erythritol, to a microorganism produced by the method, and to a method of producing erythritol using the microorganism.

Background Art

As a method of producing erythritol, there have been known a method in which the production is made by cultivating a yeast belonging to the genera Trigonopsis or Candida in a medium containing glycerol as a carbon source (Japanese Patent Publication No. Sho-47-41549), a method in which the production is made by cultivating a yeast belonging to the genera Candida, Torulopsis, or Hansenula in a medium containing a hydrocarbon or the like as a carbon source (Japanese Patent Publication No. Sho-51-21072), and the like. However, these methods have not been industrialized yet since the raw materials used as the carbon source are unsuitable for practical productions on an industrial scale.

There have also been known methods in which the production is made by cultivating a microorganism belonging to the genus Aureobasidium (Aureobasidium sp. SN-G42 (FERM P-8940)) etc.) in a medium containing a saccharide such as glucose as a carbon source (U. S. Patent Nos. 4,939,091 and 5,036,011 and the like).

Also, there have been known methods in which the production is made by cultivating Moniliella tomentosa var. pollinis in a medium containing a saccharide such as glucose as a carbon source (Japanese Patent Application Laid-open No. Sho-60-110295 and the like). While it is excellent in using glucose, which is an inexpensive and safe raw material and which has high productivity, this method is not always industrially advantageous since considerable foaming occurs during the cultivation to an extent where usually used antifoam agents are useless and, hence, addition of a large amount of expensive xanthan gum or the like is necessary.

Strains belonging to the genera Moniliella and Trichosporonoides which produce erythritol in high yields were also found to cause serious foaming during their cultivation to an extent where usually used antifoam agents were useless. In fermentative productions by microorganisms, suppression of foaming means inhibition of a decrease in productivity and an increase in the probability of contamination of saprophytic microorganisms inside an fermentation tank due to blowing-out of the culture broth and inhibition of contamination of production installation or the like by blown-out culture broth, so that it is indispensable to suppress such foaming in actual production.

Disclosure of the Invention

The present invention has been made in view of solving the problem of serious foaming occurring during aerobical cultivation of and being a defect specific to the genus Moniliella and the genus Trichosporonoides closely related thereto, that exhibit high productivity from a fermentable saccharide, which is an inexpensive and safe raw material, and to providing an inexpensive and efficient method of producing erythritol.

In order to achieve the above-described object, the present inventors have made an intensive investigation on improvement of microorganisms having the ability of producing erythritol. As a result, it has been found that removal of cell aggregates from the culture of a yeast-like filamentous fungus having the ability of producing erythritol and subsequent collection of the cells remaining in the culture enables one to efficiently obtain erythritol-producing microorganisms that will not form substantial foams during aerobic cultivation. Further, it has been found that these erythritol-producing microorganisms have physical properties such that when fractionated with water and a water-insoluble solvent, the microorganisms remain in the water layer in amounts of at least 20% and that use of the microorganisms can solve the problem of serious foaming during the cultivation. The present invention has been accomplished based on this discovery.

That is, according to the present invention, there is provided (1) a method of producing a erythritol-producing microorganism which does not form substantial foams during aerobic cultivation and which has an ability of producing erythritol, comprising the steps of: cultivating a microorganism having an ability of producing erythritol in a liquid medium, removing a microbial aggregate from the culture, and collecting a microorganism which does not form substantial foams during aerobic cultivation from the microorganism remaining in the culture.

According to a preferred aspect of the present invention, there are provided (2) a method as described in (1) above, wherein the step of cultivating a microorganism in a liquid medium and removing the microbial aggregate from the culture is repeated, (3) a method as described in (1) or (2) above, wherein the microorganism is subjected to a mutation treatment prior to the cultivation in the liquid medium.

According to a more preferred aspect of the present invention, there are provided (4) a method as described in any one of (1) to (3) above, wherein the microorganism cultivated in the liquid medium is a yeast-like filamentous fungus, (5) a method as described in (4) above, wherein the yeast-like filamentous fungus is a microorganism belonging to the genus Moniliella, (6) a method as described in (5) above, wherein the microorganism belonging to the genus Moniliella is a microorganism selected from the group consisting of Moniliella pollinis and Moniliella suaveolens var. nigra, and (7) a method as described in (5) above, wherein the microorganism belonging to the genus Moniliella is a microorganism selected from the group consisting of Moniliella pollinis CBS461.67, Moniliella pollinis MCI3554, Moniliella suaveolens var. nigra CBS223.32, Moniliella suaveolens var. nigra CBS382.36, and Moniliella suaveolens var. nigra CBS223.79.

Further, there are provided (8) a method as described in (4) above, wherein the yeast-like filamentous fungus is a microorganism belonging to the genus Trichosporonoides, (9) a method as described in (8) above, wherein the microorganism belonging to the genus Trichosporonoides is a microorganism selected from the group consisting of Trichosporonoides oedocephalis, Trichosporonoides megachiliensis, Trichosporonoides madida, Trichosporonoides nigrescens, and Trichosporonoides spathulata, and (10) a method as described in (8) above, wherein the microorganism belonging to the genus Trichosporonoides is a microorganism selected from the group consisting of Trichosporonoides oedocephalis CBS649.66, Trichosporonoides oedocephalis CBS568.85, Trichosporonoides megachiliensis CBS567.85, Trichosporonoides megachiliensis ATCC76718, Trichosporonoides madida CBS240.79, Trichosporonoides nigrescens CBS268.81, Trichosporonoides nigrescens CBS269.81, Trichosporonoides spathulata CBS241.79, Trichosporonoides spathulata CBS242.79A, and Trichosporonoides spathulata CBS242.79B.

According to another aspect (second aspect), there are provided (11) a method of producing an erythritol-producing microorganism, comprising the steps of: collecting in a liquid medium a microorganism having an ability of producing erythritol and physical properties such that when fractionated with water and a water-insoluble solvent, the microorganism remains in a water layer in an amount of at least 20% and/or a microorganism which has a hydrophobicity of 80% or less, and collecting from the microorganism(s) a microorganism which does not form substantial foams during aerobic cultivation.

According to a preferred aspect of the present invention, there are provided (12) a method as described in (11) above, further comprising the steps of: cultivating a microorganism having an ability of producing erythritol in a liquid medium, and removing a microbial aggregate from the culture, (13) a method as described in (12) above, wherein the step of cultivating a microorganism in a liquid medium and removing the microbial aggregate from the culture is repeated, and (14) a method as described in (12) or (13) above, wherein the microorganism is subjected to a mutation treatment prior to the cultivation in the liquid medium.

According to a more preferred aspect of the present invention, there are provided (15) a method as described in any one of (11) to (14) above, wherein the microorganism cultivated in the liquid medium is a yeast-like filamentous fungus, (16) a method as described in (15), wherein the yeast-like filamentous fungus is a microorganism belonging to the genus Moniliella, (17) a method as described in (16) above, wherein the microorganism belonging to the genus Moniliella is a microorganism selected from the group consisting of Moniliella pollinis and Moniliella suaveolens var. nigra, and (18) a method as described in (16) above, wherein the microorganism belonging to the genus Moniliella is a microorganism selected from the group consisting of Moniliella pollinis CBS461.67, Moniliella pollinis MCI3554, Moniliella suaveolens var. nigra CBS223.32, Moniliella suaveolens var. nigra CBS382.36, and Moniliella suaveolens var. nigra CBS223.79.

Further, there are provided (19) a method as described in (15) above, wherein the yeast-like filamentous fungus is a microorganism belonging to the genus Trichosporonoides, (20) a method as described in (19) above, wherein the microorganism belonging to the genus Trichosporonoides is a microorganism selected from the group consisting of Trichosporonoides oedocephalis, Trichosporonoides megachiliensis, Trichosporonoides madida, Trichosporonoides nigrescens, and Trichosporonoides spathulata, and (21) a method as described in (19) above, wherein the microorganism belonging to the genus Trichosporonoides is a microorganism selected from the group consisting of Trichosporonoides oedocephalis CBS649.66, Trichosporonoides oedocephalis CBS568.85, Trichosporonoides megachiliensis CBS567.85, Trichosporonoides megachiliensis ATCC76718, Trichosporonoides madida CBS240.79, Trichosporonoides nigrescens CBS268.81, Trichosporonoides nigrescens CBS269.81, Trichosporonoides spathulata CBS241.79, Trichosporonoides spathulata CBS242.79A, and Trichosporonoides spathulata CBS242.79B.

According to still another aspect (third aspect), there are provided (22) an erythritol-producing microorganism obtained by a method as described in any one of (1) to (10) above, in which microorganism does not form substantial foams during aerobic cultivation.

According to a preferred aspect of the present invention, there are provided (23) an erythritol-producing microorganism which does not form substantial foams during aerobic cultivation, the microorganism being obtained by a method as described in (7) above and selected from the group consisting of MCI3371 (FERM BP-6173) which is a mutant of Moniliella pollinis CBS461.67, MCI3555 (FERM BP-6171) which is a mutant of Moniliella pollinis MCI3554, MCI3598 which is a mutant of Moniliella suaveolens var. nigra CBS223.32, MCI3599 which is a mutant of Moniliella suaveolens var. nigra CBS382.36, and MCI3600 which is a mutant of Moniliella suaveolens var. nigra CBS223.79

Also, there is provided (24) an erythritol-producing microorganism which does not form substantial foams during aerobic cultivation, the microorganism being obtained by a method as described in (10) above and selected from the group consisting of MCI3439 (FERM BP-6308) which is a mutant of Trichosporonoides oedocephalis CBS649.66, MCI3440 (FERM BP-6175) which is a mutant of Trichosporonoides oedocephalis CBS568.85, MCI3369 (FERM BP-6172) which is a mutant of Trichosporonoides megachiliensis CBS567.85, MCI3604 which is a mutant of Trichosporonoides megachiliensis ATCC76718, MCI3441 (FERM BP-6309) which is a mutant of Trichosporonoides madida CBS240.79, MCI3437 (FERM BP-6174) which is a mutant of Trichosporonoides nigrescens CBS268.81, MCI3438 (FERM BP-6307) which is a mutant of Trichosporonoides nigrescens CBS269.81, MCI3601 which is a mutant of Trichosporonoides spathulata CBS241.79, MCI3602which is a mutant of Trichosporonoides spathulata CBS242.79A, and MCI3603 which is a mutant of Trichosporonoides spathulata CBS242.79B.

According to yet another aspect (fourth aspect) of the present invention, there is provided (25) an erythritol-producing microorganism which does not form substantial foams during aerobic cultivation, the microorganism being obtained by a method as described in any one of (11) to (21) above.

According to a further aspect (fifth aspect) of the present invention, there is provided (26) a method of producing erythritol, comprising the steps of: cultivating an erythritol-producing microorganism as described in any one of (22) to (25) above or a mutant thereof in a medium, and collecting erythritol from the culture.

According to another aspect (sixth aspect) of the present invention, there is provided (27) an erythritol-producing microorganism belonging to the genus Moniliella having an ability of producing erythritol, in which microorganism does not form substantial foams during aerobic cultivation.

According to still another aspect (seventh aspect) of the present invention, there is provided (28) an erythritol-producing microorganism belonging to the genus Moniliella having an ability of producing erythritol, in which microorganism has physical properties such that when fractionated with water and a water-insoluble solvent, the microorganism remains in a water layer in an amount of at least 20% and/or a hydrophobicity of 80% or less, and which microorganism does not form substantial foams during aerobic cultivation.

According to yet another aspect (eighth aspect) of the present invention, there is provided (29) an erythritol-producing microorganism which is a mutant of a microorganism selected from the group consisting of Moniliella pollinis and Moniliella suaveolens var. nigra, the mutant having an ability of producing erythritol and forming no substantial foam during aerobic cultivation.

According to yet still another aspect (ninth aspect) of the present invention, there is provided (30) an erythritol-producing microorganism which is a mutant of a microorganism selected from the group consisting of Moniliella pollinis CBS461.67, Moniliella pollinis MCI3554, Moniliella suaveolens var. nigra CBS223.32, Moniliella suaveolens var. nigra CBS382.36, and Moniliella suaveolens var. nigra CBS223.79, the mutant having an ability of producing erythritol and forming no substantial foam during aerobic cultivation.

According to another aspect (tenth aspect) of the present invention, there is provided (31) an erythritol-producing microorganism which is selected from the group consisting of MCI3371 (FERM BP-6173) which is a mutant of Moniliella pollinis CBS461.67, MCI3555 (FERM BP-6171) which is a mutant of Moniliella pollinis MCI3554, MCI3598 which is a mutant of Moniliella suaveolens var. nigra CBS223.32, MCI3599 which is a mutant of Moniliella suaveolens var. nigra CBS382.36, and MCI3600 which is a mutant of Moniliella suaveolens var. nigra CBS223.79, the mutant forming no substantial foam during aerobic cultivation.

According to another aspect (eleventh aspect) of the present invention, there is provided (32) an erythritol-producing microorganism belonging to the genus Trichosporonoides, having an ability of producing erythritol, in which microorganism does not form substantial foams during aerobic cultivation.

According to still another aspect (twelfth aspect) of the present invention, there is provided (33) an erythritol-producing microorganism belonging to the genus Trichosporonoides, having an ability of producing erythritol, in which microorganism has physical properties such that when fractionated with water and a water-insoluble solvent, the microorganism remains in a water layer in an amount of at least 20% and/or a hydrophobicity of 80% or less, and which microorganism does not form substantial foams during aerobic cultivation.

According to yet another aspect (thirteenth aspect) of the present invention, there is provided (34) an erythritol-producing microorganism which is a mutant of a microorganism selected from the group consisting of Trichosporonoides oedocephalis, Trichosporonoides megachiliensis, Trichosporonoides madida, Trichosporonoides nigrescens, and Trichosporonoides spathulata, the mutant having an ability of producing erythritol and forming no substantial foam during aerobic cultivation.

According to still another aspect (fourteenth aspect) of the present invention, there is provided (35) an erythritol-producing microorganism which is a mutant of a microorganism selected from the group consisting of Trichosporonoides oedocephalis CBS649.66, Trichosporonoides oedocephalis CBS568.85, Trichosporonoides megachiliensis CBS567.85, Trichosporonoides megachiliensis ATCC76718, Trichosporonoides madida CBS240.79, Trichosporonoides nigrescens CBS268.81, Trichosporonoides nigrescens CBS269.81, Trichosporonoides spathulata CBS241.79, Trichosporonoides spathulata CBS242.79A, and Trichosporonoides spathulata CBS242.79B, the mutant having an ability of producing erythritol and forming no substantial foam during aerobic cultivation.

According to a further aspect (fifteenth aspect) of the present invention, there is provided (36) an erythritol-producing microorganism selected from the group consisting of MCI3439 (FERM BP-6308) which is a mutant of Trichosporonoides oedocephalis CBS649.66, MCI3440 (FERM BP-6175) which is a mutant of Trichosporonoides oedocephalis CBS568.85, MCI3369 (FERM BP-6172) which is a mutant of Trichosporonoides megachiliensis CBS567.85, MCI3604 which is a mutant of Trichosporonoides megachiliensis ATCC76718, MCI3441 (FERM BP-6309) which is a mutant of Trichosporonoides madida CBS240.79, MCI3437 (FERM BP-6174) which is a mutant of Trichosporonoides nigrescens CBS268.81, MCI3438 (FERM BP-6307) which is a mutant of Trichosporonoides nigrescens CBS269.81, MCI3601 which is a mutant of Trichosporonoides spathulata CBS241.79, MCI3602 which is a mutant of Trichosporonoides spathulata CBS242.79A, and MCI3603 which is a mutant of Trichosporonoides spathulata CBS242.79B, the microorganism having an ability of producing erythritol and forming no substantial foam during aerobic cultivation.

Hereafter, the present invention will be described in greater detail.

Herein, by the term "substantial foams" is meant highly dense and stable foams which cannot be defoamed with any commercially available antifoam agent used in cultivation of microorganisms. By the term "a microorganism which does not form substantial foams during aerobic cultivation" is meant a microorganism which does not cause foaming due to the microorganism during aerobic cultivation and foaming due to the components of the medium or the like can be suppressed by addition of a usual antifoam agent.

In the production method of producing an erythritol-producing microorganism according to the present invention, the "microorganism having an ability of producing erythritol" used as a parent strain may be any microorganism that has an ability of producing erythritol from a fermentable saccharide used as a main carbon source, such as glucose or fructose. Usually, a yeast-like filamentous fungus is used. More specifically, there can be cited microorganisms belonging to the genus Moniliella and those belonging to the genus Trichosporonoides as preferred microorganisms.

Examples of the microorganisms belonging to the genus Moniliella include Moniliella pollinis and Moniliella suaveolens var. nigra.

Among them, preferred strains include, for example, Moniliella pollinis CBS461.67, Moniliella pollinis MCI3554 (FERM BP-6170), Moniliella suaveolens var. nigra CBS223.32, Moniliella suaveolens var. nigra CBS382.36, Moniliella suaveolens var. nigra CBS223.79, and the like.

Examples of the microorganism belonging to the genus Trichosporonoides include Trichosporonoides oedocephalis, Trichosporonoides megachiliensis, Trichosporonoides madida, Trichosporonoides nigrescens, Trichosporonoides spathulata, and the like.

Among them, preferred strains include, for example, Trichosporonoides oedocephalis CBS649.66, Trichosporonoides oedocephalis CBS568.85, Trichosporonoides megachiliensis CBS567.85, Trichosporonoides megachiliensis ATCC76718, Trichosporonoides madida CBS240.79, Trichosporonoides nigrescens CBS268.81, Trichosporonoides nigrescens CBS269.81, Trichosporonoides spathulata CBS241.79, Trichosporonoides spathulata CBS242.79A, Trichosporonoides spathulata CBS242.79B, and the like.

All these strains have been deposited at Centraal Bureau voor Schimmelcultures (CBS) in Holland and American Type Culture Collection (ATCC), both of which are international depositories and are readily available to one skilled in the art. Moniliella pollinis MCI3554 (FERM BP-6170) has been deposited since November 19, 1997 at Research Institute of Bioengineering and Industrial Technology, Institute of Industrial Science and Technology, Ministry of International Trade and Industry, Japan (1-3, Higashi 1-chome, Tsukuba-shi, Ibaraki-ken, zip code 305, Japan) with an accession number of FERM BP-6170 as an international deposition under Budapest Treaty.

In the production method of producing an erythritol-producing microorganism according to the present invention, the above-described microorganisms are cultivated in a liquid medium, a microbial aggregate or aggregates is or are removed, and then a microorganism which does not form substantial foams during aerobic cultivation is collected from the microorganisms remaining in the culture.

The cultivation is carried out in a liquid medium having the same composition as that of the medium used in the production method of producing erythritol by the microorganism of the present invention described later, preferably in an aerobic condition such as aeration, stirring, shaking, or the like. Suitable pH of the medium is usually pH 3 to 7, preferably pH 3 to 4.5, and suitable cultivation temperature is 25 to 37 °C, preferably 27 to 35 °C. Suitable cultivation time is usually 1 to 7 days, preferably 2 to 5 days.

Removal of microbial aggregate or aggregates from the culture can be performed by a method in which non-aggregated cells are separated using a micro-manipulator or the like, a method in which microbial aggregate or aggregates is or are filtered by a paper filter or the like, a method in which a cell suspension is aerated to remove a microbial aggregate or aggregates as a foam, or the like.

While the step of cultivating the above-described microorganism and removing the microbial aggregate or aggregates may be performed once, it is preferred that this step is carried out repeatedly. Repetition of the step allows for efficient collection of microorganisms which do not form substantial foams during aerobic cultivation. Suitable number of repetition is preferably 5 to 15 times.

Further, a mutational treatment of a microorganism having an ability of producing erythritol and use of the microorganism in the above-described step of cultivation and removal microbial aggregate(s) allow for a further efficient collection of microorganisms which do not form substantial foams during aerobic cultivation. As the method of mutational treatment, there can be cited usually used methods known per se, for example, such as irradiation of ultraviolet rays, irradiation of X-rays, radiation exposure, a treatment with a mutagen such as N-methyl-N'-nitro-nitrosoguanidine (NTG), artificial mutational treatments such as gene recombination and cell fusion, and the like.

Collection of microorganisms which do not form substantial foams during aerobic cultivation from the microorganisms remaining in the culture from which the microbial aggregate(s) has or have been removed may be performed, for example, by inoculating the culture from which microbial aggregate(s) has or have been removed to an agar medium having a composition similar to that of the liquid medium, separating colonies, cultivating each of the separated colonies in a liquid medium in a baffled Erlenmyer flask under aerobic conditions, and selecting a strain which forms less foam.

The microorganism which does not form substantial foams during aerobic cultivation obtained as described above may be further subjected to a mutational treatment, followed by the step of cultivating and removing microbial aggregate(s) and/or the step of separating and selecting, again.

The microorganisms of the present invention thus obtained include any microorganisms that are obtained from the microorganisms described earlier as a parent strain by the above-described methods and that do not form substantial foams during aerobic cultivation and have an ability of producing erythritol. Specifically, there can be cited yeast-like filamentous funguss, for example, microorganisms belonging to the genus Moniliella or those belonging to the genus Trichosporonoides, that have the above-described characteristics. More specifically, the following strains are cited as preferred examples.

(1) MCI3371 (FERM BP-6173), which is a mutant of Moniliella pollinis CBS461.67
(2) MCI3555 (FERM BP-6171), which is a mutant of Moniliella pollinis MCI3554,
(3) MCI3598, which is a mutant of Moniliella suaveolens var. nigra CBS223.32,
(4) MCI3599, which is a mutant of Moniliella suaveolens var. nigra CBS382.36,
(5) MCI3600, which is a mutant of Moniliella suaveolens var. nigra CBS223.79,
(6) MCI3439 (FERM BP-6308), which is a mutant of Trichosporonoides oedocephalis CBS649.66,
(7) MCI3440 (FERM BP-6175), which is a mutant of Trichosporonoides oedocephalis CBS568.85,
(8) MCI3369 (FERM BP-6172), which is a mutant of Trichosporonoides megachiliensis CBS567.85,
(9) MCI3604, which is a mutant of Trichosporonoides megachiliensis ATCC76718,
(10) MCI3441 (FERM BP-6309), which is a mutant of Trichosporonoides madida CBS240.79,
(11) MCI3437 (FERM BP-6174), which is a mutant of Trichosporonoides nigrescens CBS268.81,
(12) MCI3438 (FERM BP-6307), which is a mutant of Trichosporonoides nigrescens CBS269.81,
(13) MCI3601, which is a mutant of Trichosporonoides spathulata CBS241.79,
(14) MCI3602, which is a mutant of Trichosporonoides spathulata CBS242.79A, and
(15) MCI3603, which is a mutant of Trichosporonoides spathulata CBS242.79B.

Among the above-described strains, (1) MCI3371 strain (FERM BP-6173), (2) MCI3555 strain (FERM BP-6171), (6) MCI3439 strain (FERM BP-6308), (7) MCI3440 strain (FERM BP-6175), (8) MCI3369 strain (FERM BP-6172), (10) MCI3441 strain (FERM BP-6309), (11) MCI3437 strain (FERM BP-6174), and (12) MCI3438 strain (FERM BP-6307), have been deposited at Research Institute of Bioengineering and Industrial Technology, Institute of Industrial Science and Technology, Ministry of International Trade and Industry, Japan (1-3, Higashi 1-chome, Tsukuba-shi, Ibaraki-ken, zip code 305, Japan) with respective accession numbers in the parentheses (headed by "FERM BP-") as an international deposition under Budapest Treaty. MCI3371 strain (FERM BP-6173) and MCI3369 strain (FERM BP-6172) were deposited on November 28, 1996 with respective receipt numbers of FERM P-15967 and FERM P-15969 and transferred on November 19, 1997 to international deposition under Budapest Treaty. MCI3440 strain (FERM BP-6175) and MCI3437 strain (FERM BP-6174) were deposited on March 28, 1997 with respective receipt numbers of FERM P-16167 and FERM P-16164 and transferred on November 19, 1997 to international deposition under Budapest Treaty. MCI3555 strain has been deposited as international deposition since November 19, 1997. MCI3439 strain (FERM BP-6308), MCI3441 strain (FERM BP-6309), and MCI3438 strain (FERM BP-6307) were deposited on March 28, 1997 with respective receipt numbers of FERM P-16166, FERM P-16168, and FERM P-16165 and transferred on March 26, 1998 to international deposition under Budapest Treaty.

The above-described erythritol-producing microorganisms provided by the present invention are preferably those microorganisms which have physical properties such that when fractionated with water and a water-insoluble solvent, the microorganisms remain in the water layer in amounts of at least 20% and/or those microorganisms which have a hydrophobicity of up to 80.

Thus, according to another aspect of the present invention, there is provided a method of producing an erythritol-producing microorganism, comprising the steps of collecting a microorganism which has an ability of producing erythritol and physical properties such that when fractionated with water and a water-insoluble solvent, the microorganism remains in a water layer in an amount of at least 20% and/or a microorganism which has a hydrophobicity of 80% or less, and collecting from said microorganism(s) a microorganism which does not form substantial foams during aerobic cultivation. The hydrophobicity of the microorganism is usually 80% or less, preferably 70% or less.

As shown in Examples 4 to 18 and Comparative Examples 1 to 15 hereinbelow, microorganisms which do not form substantial foams during aerobic cultivation each have a hydrophobicity of 80% or less, which suggests some correlation between the state of forming no substantial foams during aerobic cultivation and the hydrophobicity. Therefore, there may be done either one both of selection of a microorganism which does not form substantial foams during aerobic cultivation and selection of a microorganism which has a hydrophobicity of 80% or less. When the both selections are made, either one of them may be done first.

In the method of the present invention, the selections may be done by inoculating an erythritol-producing microorganism on the above-described agar medium, for example, cultivating the microorganism, separating colonies, and subjecting a portion of the microorganisms obtained from each colony to the measurement of physical properties.

Examples of the water-insoluble solvent used in the present invention include toluene, benzene, ethyl acetate, chloroform, cyclohexane, hexanol, octanol, and propanol, preferably toluene, benzene, and octanol, and particularly preferably toluene.

Fractionation of microorganisms with water and a water-insoluble solvent may be culculated by a method usually used and known per se, for example, by preparing an aqueous suspension of a microorganism, adding a suitable amount of the above-described water-insoluble solvent thereto, stirring the mixture, and measuring the amount of the microorganism in a water layer and/or a solvent layer.

The hydrophobicity of a microorganism is a value defined by the following equation:

$$\text{Hydrophobicity} = 100 \times (1 - R/I)$$

(in the equation, R represents an optical absorbance of the water layer after treatment with a water-insoluble solvent, and I represents an optical absorbance of the water layer before the treatment.)

The optical absorbance of the water layer in the above equation can be obtained by preparing an aqueous suspension of a microorganism, measuring the optical absorbance of the aqueous suspension (for example, at 660 nm) using a spectrophotometer, adding a water-insoluble solvent to the suspension, stirring the mixture, separating a water layer and a water-insoluble solvent layer from each other, and then measuring the optical absorbance of the water layer at the same wavelength as above. The aqueous suspension of a microorganism is not limited particularly as far as there exists a microorganism of which measurement of optical absorbance is possible. Suitably, it is prepared such that its optical absorbance is usually 0.1 to 1.0, preferably 0.3 to 0.6. The amount of water-insoluble solvent to be added is not limited particularly but it is preferred to add it in the same amount as the aqueous suspension of the microorganism. The mixing of the aqueous suspension of a microorganism with the water-insoluble solvent may be performed using a test tube mixer or the like. It is preferred that microorganisms be subjected to washing, if desired before preparation of the aqueous suspension. Further, collection of the microorganism which does not form substantial foams during aerobic cultivation may be done by the above-described method. Note that in the present invention, optical absorbance can be replaced by turbidity.

The selection of microorganisms by the above-described physical properties may be done in combination with the steps of cultivating erythritol-producing microorganisms in a liquid medium and removing microbial aggregate(s) from the culture. That is, the selection may be carried out by carrying out the step in combination before or after the selection of microorganisms by the above-described physical properties. Further, the cultivation of microorganisms in a liquid culture may be performed after the above-described mutational treatment.

In this manner, according to the method of the present invention, there is provided an erythritol-producing microorganism which has an ability of producing erythritol and which does not form substantial foams during aerobic cultivation.

Next, in order to elucidate the taxonomical classification of the erythritol-producing microorganisms provided by the present invention, the results (mycological properties) of identification tests which the present inventors conducted on microorganisms are shown below.

## Identification of MCI3369 (FERM BP-6172) strain

MCI3369 strain after cultivation on PDA (potato dextrose agar) at 24 °C appeared at first white, and turned afterward to olivy grey or olive brown in the case of older cultures of 2 weeks or more. The fungus grew at rapidly and proliferated by yeast-like budding. The yeast-like cells were colorless at first and then turned olivish brown. Vegetative hyphae, which developed well, with septa and branches, had a width of 2 to 3.8μm, were at first colorless, and afterwards had slightly thickened membrane and turned brown. Development of aerial hyphae was excellent and budding-type conidia were formed on the side of aerial hyphae. Vegetative hyphae and aerial hyphae were cut into fragments to form arthrospore-like conidia. The arthrospores were cylindrical or barrel-form (3.6 to 25 $\mu$m X 2.2 to 4.3 $\mu$m), at first colorless and turned pale brown afterwards. The budding-type conidia were single or made a chain consisting of 3 to 4 conidia. The conidia were of an oblong ellipse, with a size of 3.4 to 7.5 $\mu$m X 1.9 to 4.1 $\mu$m (average 6.5±1.2 $\mu$m X 3.8±0.6 $\mu$m) and appeared at first colorless and turned olivy brown afterwards.

The morphological properties of the instant strain (MCI3369) well coincided with the characteristics of the type strain of Trichosporonoides megachiliensis CBS567.85, a parent strain of MCI3369. Therefore, this strain was identified as Trichosporonoides megachiliensis.

Identification of MCI3371 (FERM BP-6173) strain

MCI3371 strain after cultivation on PDA (potato dextrose agar) at 24 °C appeared at first white to yellowish white, and turned dull yellow after cultivation for 1 week or blackish brown in the case of older cultures. The fungus grew at rapidly and proliferated by yeast-like budding. The budding cells at first had a thin membrane and appeared olivish brown and afterwards had a thickened membrane and colored. Simultaneously with the yeast-like budding, vegetative hyphae elongated. The vegetative hyphae had septa and branched. They had a width of 2 to 4.5$\mu$m, were at first colorless and turned brown afterwards. The hyphae were cut into fragments to form arthrospore-like conidia or budding-type conidia were formed on the side or top of the hypha. The arthrospores were cylindrical or barrel-form (6 to 35 $\mu$m X 2.5 to 5.0 $\mu$m), at first colorless and turned pale brown afterwards. The budding-type conidia were single or made a chain consisting of 2 to 3 conidia. The conidia were oval to elliptical, or spheroidal, with a size of 4.7 to 9.4 $\mu$m X 3.1 to 5.6 $\mu$m (average 6.8±1.3 $\mu$m X 4.5±0.6 $\mu$m) and appeared at first colorless and turned olivy brown afterwards.

The strain (MCI3371) had characteristics 1) that it had a dimorphism, i.e., arthrospore and budding-type conidium, 2) that the budding-type conidia were formed acropetally but not synchronously, and so on. Based on these characteristics, retrieval of genera was conducted according to the monograph of De Hoog & Hermanides-Nijhof (1977), which confirmed that the instant strain belonged to the genus Moniliella. According to De Hoog, "The Black Yeasts, II: Moniliella and Allied Genera", Studies in Mycology No. 19, 1-90 (1979), Moniliera is known to include 3 species and 2 varieties: Moniliella suaveolens var. suaveolens, Moniliella suaveolens var. niger, Moniliella acetoabutens, and Moniliella pollinis. These species and varieties are distinguished mainly by the morphological characteristics of budding-type conidia and arthrospores. As a result of detailed study of the morphological properties of the present strain, it was found that this strain well coincided with the description of Moniliella pollinis. Therefore, this strain was identified as Moniliella pollinis.

Identification of MCI3437 (FERM BP-6174) strain

MCI3437 strain is a mutant derived from Trichosporonoides nigrescens CBS268.81, which after cultivation on LCA (Miura medium) at 24 °C appeared at first white to yellowish white and turned yellowish brown after cultivation for 1 week or dark yellowish brown in the case of older cultures of 2 weeks or more. The fungus grew at moderately and proliferated by yeast-like budding. The budding cells at first had a thin membrane and appeared olivish brown and afterwards had a thickened membrane and colored blackish brown, proliferating by multipolar budding. Proliferation occurred by budding once to 3 or 4 times. Simultaneously with the yeast-like budding, basal hyphae elongated. The basal hyphae had septa and branched. They had a width of 2 to 4.5 $\mu$m, were at first colorless and turned brown afterwards. The hyphae were cut into fragments to form arthrospore-like conidia or budding-type conidia were formed on the side or top of the hypha. The arthrospores were cylindrical or barrel-form of various lengths, with a width of 2.5 to 5.0 $\mu$m, at first colorless and turned brown afterwards. The budding-type conidia, formed on the side or top of the basal hypha, were alone or made a chain consisting of 2 to 3 conidia. The conidia were spheroidal to elliptical (4.3 to 9.2 $\mu$m X 3.8 to 6.5 $\mu$m), brown and the membrane became thickened. The strain did not grow at 37 °C.

The strain (MCI3437) had characteristics 1) that it had a dimorphism, i.e., arthrospore and budding-type conidium, 2) that the budding-type conidia were formed acropetally but not synchronously, and so on. Based on these characteristics, comparison was made with a parent strain of Trichosporonoides nigrescens and retrieval of genera and species was conducted according to the monograph of G. S. de Hoog (1979) and the original description by A. D. Hocking & J. I. Pitt (1981), with the result that the properties of the present strain well coincided with the description of the parent strain of Trichosporonoides nigrescens. Therefore, this strain was identified as Trichosporonoides nigrescens.

Identification of MCI3438 (FERM BP-6307) strain

MCI3438 strain is a mutant derived from Trichosporonoides nigrescens CBS269.81, which after cultivation on LCA (Miura medium) at 24 °C appeared at first white to yellowish white and turned yellowish brown after cultivation for 1 week or dark yellowish brown in the case of older cultures of 2 weeks or more. The fungus grew at moderately and proliferated by yeast-like budding. The budding cells at first had a thin membrane and appeared olivish brown and afterwards had a thickened membrane and colored blackish brown, proliferating by multipolar budding. Proliferation occurred by budding once to 3 or 4 times. Simultaneously with the yeast-like budding, basal hyphae elongated. The basal hyphae had septa and branched. They had a width of 2 to 4.5 $\mu$m, were at first colorless and turned brown afterwards. The hyphae were cut into fragments to form arthrospore-like conidia or budding-type conidia were formed on the side or top of the hypha. The arthrospores were cylindrical or barrel-form of various lengths, with a width of 2.5 to 5.0 $\mu$m, at first colorless and turned brown afterwards. The budding-type conidia, formed on the side or top of the basal hypha, were single or made a chain consisting of 2 to 3 conidia. The conidia were spheroidal to elliptical (3.4 to 9.8 $\mu$m X 3.8 to 6.3 $\mu$m), at first colorless and turned brown afterwards and the membrane became thickened. The strain did not grow at 37 °C.

The strain (MCl3438) had characteristics 1) that it had a dimorphism, i.e., arthrospore and budding-type conidium, 2) that the budding-type conidia were formed acropetally but not synchronously, and so on. Based on these characteristics, comparison was made with a parent strain of Trichosporonoides nigrescens and retrieval of genera and species was conducted according to the original descriptions by G. S. de Hoog (1977) and A. D. Hocking & J. I. Pitt (1981), respectively, with the result that the properties of the present strain well coincided with the description of the parent strain of Trichosporonoides nigrescens. Therefore, this strain was identified as Trichosporonoides nigrescens.

Identification of MCl3439 (FERM BP-6308) strain

MCl3439 strain is a mutant derived from Trichosporonoides oedocephalis CBS649.44, which after cultivation on LCA (Miura medium) at 24 °C appeared at first white to yellowish white and turned brown after cultivation for 1 week or dark yellowish brown in the case of older cultures of 2 weeks or more. The fungus grew rapidly and proliferated by yeast-like budding. The budding cells were colorless, proliferating by multipolar budding. Proliferation occurred by budding once to 3 or 4 times. Simultaneously with the yeast-like budding, basal hyphae and aerial hyphae elongated. The basal hyphae and aerial hyphae had septa and branched. They had a width of 2 to 4.5 $\mu$m and were colorless. The hyphae were cut into fragments to form arthrospore-like conidia or budding-type conidia were formed on the side or top of the hypha. Also, conidiophores developed from the basal hyphae, with their top swelling to form conidial heads. The conidial heads were 8.8 to 12.5 $\mu$m in diameter, and budding-type conidia were formed therefrom synchronously. The arthrospores were cylindrical or barrel-form of various lengths, with a width of 2.8 to 5.0 $\mu$m, at first colorless. The budding-type conidia, formed on the side or top of the basal hypha, were alone or made a chain consisting of 2 to 3 conidia, elliptical (4.4 to 6.3 $\mu$m X 2.2 to 3.8 $\mu$m), and colorless. The conidia formed in the conidial head were spheroidal to spherical (4.0 to 6.3 $\mu$m) and reddish brown and the strain grew at 37 °C.

The strain (MCl3439) had characteristics 1) that it had a dimorphism, i.e., arthrospore and budding-type conidium, 2) that the budding-type conidia were formed on the basal hyphae and aerial hyphae acropetally or formed synchronously from the conidial head, and so on. Based on these characteristics, comparison was made with a parent strain of Trichosporonoides oedocephalis and retrieval of genera and species was conducted according to the monograph of G. S. de Hoog (1979) and the original description by R. H. Haskins & J. F. T. Spencer (1966), with the result that the properties of the present strain well coincided with the description of the parent strain of Trichosporonoides oedocephalis. Therefore, this strain was identified as Trichosporonoides oedocephalis.

Identification of MCl3440 (FERM BP-6175) strain

MCl3440 strain is a mutant derived from Trichosporonoides oedocephalis CBS568.85, which after cultivation on LCA (Miura medium) at 24 °C appeared at first white to yellowish white and turned brown after cultivation for 1 week or dark yellowish brown in the case of older cultures of 2 weeks or more. The fungus grew at high rates and proliferated by yeast-like budding. The budding cells were colorless, proliferating by multipolar budding. Proliferation occurred by budding once to 3 or 4 times. Simultaneously with the yeast-like budding, basal hyphae and aerial hyphae elongated. The basal hyphae and aerial hyphae had septa and branched. They had a width of 2 to 4.5 $\mu$m and were colorless. The hyphae were cut into fragments to form arthrospore-like conidia or budding-type conidia were formed on the side or top of the hypha. On LCA (Miura medium) and PDA (potato dextrose agar) no conidial head was formed. The arthrospores were cylindrical or barrel-form of various lengths, with a width of 2.8 to 5.0 $\mu$m, colorless. The budding-type conidia, formed on the side or top of the basal hypha, were single or made a chain consisting of 2 to 3 conidia, elliptical (4.7 to 8.1 $\mu$m X 2.5 to 3.4 $\mu$m), colorless and the strain grew at 37 °C.

The strain (MCl3440) had characteristics 1) that it had a dimorphism, i.e., arthrospore and budding-type conidium, 2) that the budding-type conidia were formed on the basal hyphae and aerial hyphae acropetally, and so on. This strain did not form any Oedocephalis-type conidial head. According to the original description by Haskins & Spencer (1966), Trichosporonoides oedocephalis is distinguished from other species (T. spathulata, T. nigrescens, T. madida, and T. megachiliensis) mainly by its having conidial heads. The mutant differed from T. oedocephalis in this point. However, comparison was made with a parent strain of Trichosporonoides oedocephalis and retrieval of genera and species was conducted according to the monograph of G. S. de Hoog (1979) and the original description by R. H. Haskins & J. F. T. Spencer (1966), with the result that the morphological properties of the instant strain well coincided with the description of the parent strain of <u>Trichosporonoides</u> <u>oedocephalis</u> except for the lack of Oedocephalis-type conidial head. Therefore, this strain was temporarily identified as <u>Trichosporonoides</u> <u>oedocephalis</u>.

## Identification of MCI3441 (FERM BP-6309) strain

MCI3441 strain is a mutant derived from <u>Trichosporonoides</u> <u>madida</u> CBS240.79, which after cultivation on LCA (Miura medium) at 24 °C appeared at first white to yellowish white and turned brown in the case of older cultures of 2 weeks or more. The fungus grew moderately and proliferated by yeast-like budding. The budding cells were colorless, proliferating by multipolar budding. Proliferation occurred by budding once to 3 or 4 times. Simultaneously with the yeast-like budding, basal hyphae elongated with their development being poor. The basal hyphae had septa and branched with a width of 2 to 3.5 $\mu$m and were colorless. The hyphae were cut into fragments to form arthrospore-like conidia or budding-type conidia were formed on the side or top of the hypha. The arthrospores were cylindrical or barrel-form of various lengths, with a width of 2.5 to 40 $\mu$m, colorless. The budding-type conidia, formed on the side or top of the basal hypha, were single or made a chain consisting of 2 to 3 conidia, spheroidal to elliptical (3.1 to 7.8 $\mu$m X 2.8 to 4.4 $\mu$m), colorless and the strain grew at 37 °C.

The strain (MCI3441) had characteristics 1) that it had a dimorphism, i.e., arthrospore and budding-type conidium, 2) that the budding-type conidia were formed on the basal hyphae acropetally but not synchronously, and so on. Based on these characteristics, comparison was made with a parent strain of <u>Trichosporonoides</u> <u>madida</u> and retrieval of genera and species was conducted according to the monograph of G. S. de Hoog (1979), with the result that the properties of the present strain well coincided with the description of the parent strain of <u>Trichosporonoides</u> <u>madida</u>. Therefore, this strain was identified as <u>Trichosporonoides</u> <u>madida</u>.

## Identification of MCI3554 (FERM BP-6170) strain

This strain is a microorganism isolated from a dead stem of a plant in the soil and is a parent strain of MCI3555 strain of the present invention.

1) Morphological characteristics:

The colony after cultivation on PDA (potato dextrose agar) at 24 °C appeared at first white to yellowish white and turned yellowish brown after cultivation of 1 week and dark yellowish brown in the case of older cultures of 2 weeks or more. The fungus grew moderately and proliferated by yeast-like budding. The budding cells were at first colorless, had a slightly thickened membrane, turned pale brown, and were elliptical, oval or spheroidal (3.8 to 6.3 $\mu$m X 3.0 to 5.0 $\mu$m). Proliferation occurred by budding once to 3 or 4 times, with the budding being multipolar. Simultaneously with the yeast-like budding, basal hyphae and aerial hyphae elongated. The basal hyphae and aerial hyphae were of a width of 2.2 to 3.5 $\mu$m, had septa and branched, and were at first colorless and turned brown afterwards. The hyphae were cut into fragments to form arthrospore-like conidia and budding-type conidia were formed on the side or top of the hypha. The arthrospores were cylindrical or barrel-form of various lengths (9.4 to 18.8 $\mu$m X 3.1 to 4.1 $\mu$m), at first colorless and turned brown afterwards. The budding-type conidia, formed on the side and top of the basal hypha, were single or made a chain consisting of 2 to 3 conidia, spheroidal to elliptical (5.9 to 10.9 $\mu$m X 3.8 to 5.9 $\mu$m), at first colorless and turned brown and the membrane became slightly thickened.

2) Physiological characteristics:

Growth temperature: 9 to 37 °C (on PDA, 10 days' cultivation)
Optimal Growth temperature: 27 to 30 °C
Growth pH: 4 to 9 (on LCA liquid medium, 10 days' cultivation)
Optimal growth pH: 5 to 6
Utilization of carbon sources (as shown in Table 1 below)
Fermentability from sugars (as shown in Table 2 below)
Utilization of nitrogen sources (as shown in Table 3 below)

3) Taxonomical consideration

The strain (MCI3554) had characteristics 1) that it had a yeast-like budding-type cell, 2) that it had a dimorphism, i.e., arthrospore and budding-type conidium, 3) that the budding-type conidia were formed acropetally but not synchronously, and so on. Based on these characteristics, retrieval of genera and species was conducted according to the reference list in the monograph of G. S. de Hoog (1979), "The Black Yeasts, II: Moniliella and Allied Genera", Studies in Mycology No. 19, p. 1-36 and the description on the species belonging to the genus Moniliella in G. S. de Hoog & E. Gueho (1984), "Deoxyribonucleic acid base composition and taxonomy of Moniliella and allied genera", Antonie van Leeuwenhook, 135-141, with the result that the properties of the present strain well coincided with the description of the parent strain of Moniliella pollinis. Further, comparison made with a type strain of Moniliella pollinis (CBS 461.67) confirmed that the properties of this strain well coincided with those of the type strain. Therefore, this strain was identified as Moniliella pollinis.

Identification of MCI3555 (FERM BP-6171) strain

1) Morphological characteristics

The strain (MCI3555 strain) is a mutant derived from Moniliella pollinis MCI3554, whose colony after cultivation on PDA (potato dextrose agar) at 24 °C appeared at first white to yellowish white, and turned yellowish brown after cultivation for 1 week and dark yellowish brown in the case of older cultures of 2 weeks or more. The fungus grew at medium rates and proliferated by yeast-like budding. The budding cells were at first colorless with the membrane becoming slightly thickened and turned pale brown afterwards, and were elliptical, oval or spheroidal (4.0 to 7.8 $\mu$m X 3.5 to 6.2 $\mu$m). Proliferation occurred by budding once to 3 or 4 times, with the budding being multipolar. Simultaneously with the yeast-like budding, basal hyphae and aerial hyphae elongated. The basal hyphae and aerial hyphae were of a width of 1.3 to 4.1 $\mu$m, had septa and branched, and were at first colorless and turned brown afterwards. The hyphae were cut into fragments to form arthrospore-like conidia and budding-type conidia were formed on the side or top of the hypha. The arthrospores were cylindrical or barrel-form of various lengths (13.4 to 32.8 $\mu$m X 2.5 to 4.1 $\mu$m), at first colorless and turned brown afterwards. The budding-type conidia, formed on the side and top of the basal hypha, were alone or made a chain consisting of 2 to 3 conidia, spheroidal to elliptical (5.0 to 9.4 $\mu$m X 4.4 to 6.3 $\mu$m), at first colorless and turned brown and the membrane became slightly thickened.

2) Physiological characteristics:

Growth temperature: 9 to 37 °C (on PDA, 10 days' cultivation)
Optimal Growth temperature: 27 to 30 °C
Growth pH: 4 to 9 (on LCA liquid medium, 10 days' cultivation)
Optimal growth pH: 5 to 6
Utilization of carbon sources (as shown in Table 1 below)
Fermentability from sugars (as shown in Table 2 below)
Utilization of nitrogen sources (as shown in Table 3 below)

3) Taxonomical consideration

The strain (MCI3555) had characteristics 1) that it had a yeast-like budding-type cell, 2) that it had a dimorphism, i.e., arthrospore and budding-type conidium, 3) that the budding-type conidia were formed acropetally but not synchronously, and so on. Based on these characteristics, retrieval of genera and species was conducted according to the reference list in the monograph of G. S. de Hoog (1979), "The Black Yeasts, II: Moniliella and Allied Genera", Studies in Mycology No. 19, p. 1-36 and the description on the species belonging to the genus Moniliella in G. S. de Hoog & E. Gueho (1984), "Deoxyribonucleic acid base composition and taxonomy of Moniliella and allied genera", Antonie van Leeuwenhook, 135-141, with the result that the properties of the present strain well coincided with the description of the parent strain of Moniliella pollinis. Further, comparison made with a type strain of Moniliella pollinis (CBS461.67) confirmed that the properties of this strain well coincided with those of the type strain. Therefore, this strain was identified as Moniliella pollinis.

## Table 1  Utilization of Carbon Sources

| | Carbon Source | MCI3554 | MCI3555 |
|---|---|---|---|
| 1 | D-Glucose | + | + |
| 2 | D-Galactose | V | − |
| 3 | L-Sorbose | ± | − |
| 4 | D-Glucosamine | − | − |
| 5 | D-Ribose | V | ± |
| 6 | D-xylose | ± | − |
| 7 | L-Arabinose | ± | ± |
| 8 | D-Arabinose | − | − |
| 9 | L-Rhamnose | − | − |
| 10 | Sucrose | + | + |
| 11 | Maltose | + | + |
| 12 | α,α-Trehalose | − | − |
| 13 | Methyl-α-D-glucoside | − | − |
| 14 | Cellobiose | + | − |
| 15 | Salicin | − | − |
| 16 | Albutin | + | + |
| 17 | Melibiose | − | − |
| 18 | Lactose | − | − |
| 19 | Raffinose | − | − |
| 20 | Meleditose | − | − |
| 21 | Inulin | − | − |
| 22 | Soluble starch | − | − |
| 23 | Glycerin | + | + |
| 24 | meso-Erythritol | + | + |
| 25 | Ribitol | − | − |
| 26 | Xylitol | + | ± |
| 27 | L-Arabinitol | − | − |
| 28 | D-Glucitol | − | − |
| 29 | D-Mannitol | + | + |
| 30 | Galactitol | − | − |
| 31 | myo-Inositol | − | − |
| 32 | Glucono δ-lactone | ± | ± |

## Table 1  Utilization of Carbon Sources (continued)

| Carbon Source | MCI3554 | MCI3555 |
|---|---|---|
| 33  D-Gluconic acid | − | − |
| 34  D-Glucuronic acid | − | − |
| 35  D-Galacturonic acid | − | − |
| 36  DL-Lactic acid | − | − |
| 37  Succinic acid | ± | ± |
| 38  Citric acid | ± | ± |
| 39  Methanol | − | − |
| 40  Ethanol | + | + |

+: Utilized,          ±: Unclear,
V: not determinable,  −: not utilized

### Table 2

| Fermentability from sugars | | |
|---|---|---|
| Sugar | MCI3554 | MCI3555 |
| 1 D-Glucose | + | + |
| 2 D-Galactose | - | - |
| 3 Maltose | + | + |
| 4 Sucrose | + | + |
| 5 Lactose | - | - |
| 6 Raffinose | - | - |

+: Fermented,
-: Not fermented

### Table 3

| Utilization of Nitrogen Sources | | |
|---|---|---|
| Nitrogen Source | MCI3554 | MCI3555 |
| 1 Ammonium sulfate | + | + |
| 2 Potassium nitrate | + | + |
| 3 L-Lysine | + | + |
| 4 Cadaverine | + | + |

+: Utilized -: Not utilized

Using the erythritol-producing microorganisms, a method of producing erythritol according to another aspect of the present invention.

In the present invention, the above-described erythritol-producing microorganisms and mutants thereof are cultivated in media containing fermentable saccharides as main carbon sources and erythritol is collected from the cultures.

As the erythritol-producing microorganisms, there are used those described above and preferred microorganisms used in the present invention are those described as preferred ones hereinabove. Further, these strains may be mutants of which various properties such as erythritol productivity have been improved. These mutants may be bred by a known method usually used.

The medium used in the present invention may be a liquid medium comprising water having dissolved therein a carbon source, a nitrogen source, optionally inorganic salt(s), a growth factor, and the like.

As a main carbon source used in the cultivation of the above-described microorganisms, there are utilized fermentable saccharides such as glucose, fructose, glycerol, and the like. These main carbon sources may be used alone or in combination. The concentration to be used is not limited particularly but it is advantageous that the concentration is as high as possible within the ranges where production of erythritol is not inhibited. Preferred concentration is within the ranges of 20 to 60% (W/V). Also, main carbon sources may be added to the culture in portions during the cultivation. Erythritol is produced from these main carbon sources by the microorganisms used in the present invention.

As the nitrogen source used in the cultivation of microorganisms, there can be used various organic and inorganic nitrogen compounds such as ammonia salts, urea, peptone, microorganism extracts, and corn steep liquor. As the inorganic salt, there can be used various phosphoric acid salts, sulfuric acid salts, and salts of a metal such as magnesium, potassium, manganese, iron, or zinc. Also, as a growth factor, there can be added, if desired, one or more factors which promote the growth of microorganisms, such as vitamins, nucleotides, and amino acids. Although the microorganisms used in the present invention do not form substantial foams during the cultivation, it is preferred that a suitable amount of commercially available defoaming agent be added to the medium in order to prevent foaming during the cultivation due to the components contained in the medium.

Upon cultivation, microbial cells may be inoculated to a main medium directly from a slant culture. However, it is preferred to inoculate a preculture obtained by cultivation in a liquid medium for 1 to 4 days to the main medium.

The pH of the medium at initial stage of cultivation is usually pH 3 to 7, preferably pH 3 to 4.5. The cultivation temperature is suitably 25 to 37 °C, preferably 27 to 35 °C. It is preferred that the cultivation be run under aerobic conditions such as aeration, stirring, or shaking. The cultivation time preferably lasts up to consumption of the main carbon source(s) and usually the cultivation is run for 3 to 8 days. The amount of erythritol thus produced in the culture medium can be determined by a known method usually used such as gas chromatography, or high performance liquid chromatography. While it is expected that the above-described cultivation conditions may vary depending on the microorganism to be used, preferable conditions can be found by conducting preliminary experimentation in which the conditions are varied stepwise for respective microorganisms to be used.

The erythritol which was accumulated in the culture solution is separated from the culture and purified by a conventional manner. More specifically, the separation and purification can be carried out by removing solids by centrifugation, filtration or the like, decolorizing and then desalting the residual solution with activated carbon or ion exchange resin, and crystallizing erythritol from the solution.

Best Mode for Carrying Out the Invention

Hereafter, the present invention will be described in more detail by examples. However, the present invention is not limited thereto.

Example 1

Moniliella pollinis CBS461.67 was cultivated in a medium containing 1.5% of yeast extracts and 30% of glucose and the microbial cells were collected, washed twice with physiological saline and then subjected to mutational treatment with physiological saline containing 1 mg/ml of NTG at 30 °C for 60 minutes. Then, the cells were collected and suspended in the medium and incubated with shaking at 30 °C to stabilize the mutation. Subsequently, a portion of the suspension was inoculated in the medium and incubated for 2 days and then microbial aggregates were removed and the remaining cell suspension was inoculated to a concentrated medium. After repeating this procedure 7 times, the cells were spread on an agar medium of the same composition as the agar medium to allow colony formation. The cells obtained from each colony were cultivated with shaking in a baffled Erlenmyer flask and a strain which did not form foams was selected to obtain MCI3371 strain (FERM BP-6173).

In the same manner as above, MCI3600 strain and MCI3440 strain (FERM BP-6175) were obtained from Moniliella suaveolens var. nigra CBS223.79 and Trichosporonoides oedocephalis CBS568.85, respectively.

Example 2

Trichosporonoides megachiliensis CBS567.85 was incubated in a medium containing 1.5% of yeast extracts and 30% of glucose and the microbial cells were collected, washed twice with physiological saline and then subjected to mutational treatment by irradiation of ultraviolet rays for 60 minutes. Then, the cells were collected and suspended in the same medium and incubated with shaking at 30 °C to stabilize the mutation. Subsequently, a portion of the suspension was inoculated in the same medium and incubated for 2 days and then microbial aggregates were removed and the remaining cell suspension was inoculated to a concentrated medium. After repeating this procedure 3 times, the cells were spread on an agar medium of the same composition as the liquid medium to allow colony formation. The cells obtained from each colony were cultivated with shaking in a baffled Erlenmyer flask and a strain which did not form foams was selected to obtain MCl3369 strain (FERM BP-6172).

In the same manner as above, MCl3604 strain, MCl3439 strain (FERM BP-6308), MCl3437 strain (FERM BP-6174), MCl3438 (FERM BP-6307), MCl3601 strain, MCl3602 strain, MCl3603 strain, MCl3598 strain, MCl3599 strain, and MCl3555 strain (FERM BP-6171) were obtained from Trichosporonoides megachiliensis ATCC76718, Trichosporonoides oedocephalis CBS649.66, Trichosporonoides nigrescens CBS268.81, Trichosporonoides nigrescens CBS269.81, Trichosporonoides spathulata CBS241.79, Trichosporonoides spathulata CBS242.79A, Trichosporonoides spathulata CBS241.79B, Moniliella suaveolens var. nigra CBS223.32, Moniliella suaveolens var. nigra CBS382.36, and Moniliella pollinis MCl3554 (FERM BP-6170), respectively.

Example 3

Trichosporonoides madida CBS240.79 was incubated in a medium containing 1.5% of yeast extracts and 30% of glucose and microbial aggregates were removed. The remaining cell suspension was inoculated in the same medium after concentration and incubated again. After repeating this procedure 5 times, the cells were spread on an agar medium of the same composition to allow colony formation. The cells obtained from each colony were cultivated with shaking in a baffled Erlenmyer flask and a strain which did not form foams was selected therefrom to obtain MCl3441 strain (FERM BP-6309).

Examples 4 to 18

A medium (5 ml) containing 30% (W/V) of glucose and 1.0% of yeast extracts charged in test tubes of 21 mm in diameter with a cotton plug were sterilized at 120 °C for 20 minutes. MCl3437 strain (FERM BP-6174), MCl3438 strain (FERM BP-6307), MCl3439 strain (FERM BP-6308), MCl3440 strain (FERM BP-6175), MCl3369 strain (FERM BP-6179), MCl3441 strain (FERM BP-6309), MCl3371 strain (FERM BP-6173), MCl3555 strain (FERM BP-6171), MCl3598 strain, MCl3599 strain, MCl3600 strain, MCl3601 strain, MCl3602 strain, MCl3603 strain, and MCl3604 strain were inoculated in the media respectively, and cultivated with shaking at 30 °C for 3 days. Each 1 ml of the culture solution was inoculated in a 200 ml baffled Erlenmeyer flask containing 20 ml of the same medium as above, and incubated with shaking at 30 °C for 4 days. After completion of the incubation, the concentration of erythritol in the culture solution was measured by high performance liquid chromatography.

Further, using the cells incubated in the test tubes, the hydrophobicity of cells was measured by the method of Iimura et al. (Y. Iimura, S. Hara and K Otsuka, Agric. Biol. Chem., 44(4), 1215-1222, (1980)). That is, the cells washed twice with distilled water were suspended in water such that its optical absorption ($A_{660}$) was 0.6 and the same amount of toluene was added to the suspension followed by stirring. After standing the mixture for 30 minutes, the optical absorption ($A_{660}$) of the water layer was measured and the hydrophobicity (HD value) of the cells was calculated by the following equation.

$$\text{HD value} = 100 \times (1 - R/I)$$

I: Optical absorption ($A_{660}$) before the treatment with toluene,
R: Optical absorption ($A_{660}$) of the water layer after the treatment with toluene.

As a result, each strain did not show substantial foams during the cultivation. Further, each strain had a yield of erythritol and hydrophobicity as shown in Table 5.

Table 5

| Example Number | Strain | Yield of Erythritol | Hydrophobicity (HD value) |
|---|---|---|---|
| 4 | MCI3437 | 124.7 g/L | 52.0 |
| 5 | MCI3438 | 70.0 g/L | 21.2 |
| 6 | MCI3439 | 70.6 g/L | 37.7 |
| 7 | MCI3440 | 103.5 g/L | 48.8 |
| 8 | MCI3369 | 110.8 g/L | 42.3 |
| 9 | MCI3441 | 97.6 g/L | 44.0 |
| 10 | MCI3371 | 129.5 g/L | 70.0 |
| 11 | MCI3555 | 134.4 g/L | 27.2 |
| 12 | MCI3598 | 79.3 g/L | 53.2 |
| 13 | MCI3599 | 19.9 g/L | 55.5 |
| 14 | MCI3600 | 131.9 g/L | 60.3 |
| 15 | MCI3601 | 18.7 g/L | 40.2 |
| 16 | MCI3602 | 19.0 g/L | 24.5 |
| 17 | MCI3603 | 11.5 g/L | 28.0 |
| 18 | MCI3604 | 87.0 g/L | 35.2 |

Comparative Examples 1 to 15

In the same manner as in Examples 4 to 18, respective parent strains before obtaining mutants were used as they were to produce erythritol. As a result, each strain showed serious foaming during the cultivation. Each strain had a yield of erythritol and hydrophobicity as shown in Table 6.

Table 6

| Comparative Example Number | Strain | Yield of Erythritol | Hydrophobicity (HD value) |
|---|---|---|---|
| 1 | CBS268.81 | 104.7 g/L | 93.7 |
| 2 | CBS269.81 | 65.6 g/L | 93.0 |
| 3 | CBS649.66 | 58.8 g/L | 87.2 |
| 4 | CBS568.85 | 100.0 g/L | 97.6 |
| 5 | CBS567.85 | 112.9 g/L | 97.6 |
| 6 | CBS240.79 | 131.9 g/L | 85.0 |
| 7 | CBS461.67 | 97.6 g/L | 88.0 |
| 8 | MCI3554 | 117.7 g/L | 89.5 |
| 9 | CBS223.32 | 75.3 g/L | 89.1 |
| 10 | CBS382.36 | 71.8 g/L | 85.2 |
| 11 | CBS223.79 | 89.8 g/L | 90.2 |
| 12 | CBS241.79 | 6.6 g/L | 85.3 |
| 13 | CBS242.79A | 20.3 g/L | 87.0 |

Table 6 (continued)

| Comparative Example Number | Strain | Yield of Erythritol | Hydrophobicity (HD value) |
|---|---|---|---|
| 14 | CBS242.79B | 19.5 g/L | 89.0 |
| 15 | ATCC6718 | 78.9 g/L | 91.0 |

Example 19

A liquid medium (100 ml) containing 30% (W/V) of glucose and 1.0% of yeast extracts (manufactured by Asahi Beer Co., Ltd.) charged in a 500 ml Erlenmeyer flask was sterilized at 120 °C for 20 minutes. A loopful of MCI3369 strain (FERM BP-6172) slant-cultivated by a conventional method was inoculated in the medium and cultivated with shaking at 35 °C for 3 days. The culture medium (100 ml) was inoculated in a 5-liter fermentation tank charged with 3 liters of a liquid medium containing 40% (W/V) of glucose, 1.5% of yeast extracts (manufactured by Asahi Beer Co., Ltd.), and 500 PPM of a antifoam agent CA330 (manufactured by Nippon Yushi Co., Ltd.) and cultivated under conditions of 35 °C, air flow rate of 0.5 vvm, and rotation number of 700 rpm, for 4 days. As a result of measurement of erythritol concentration in the culture solution by high performance liquid chromatography, it was found that 180.9 g/L of erythritol was accumulated. During the cultivation, no foaming was observed.

Example 20

A liquid medium (100 ml) containing 30% (W/V) of glucose and 1.0% of yeast extracts (manufactured by Asahi Beer Co., Ltd.) charged in a 500 ml Erlenmeyer flask with a cotton plug was sterilized at 120 °C for 20 minutes. A loopful of MCI3771 strain (FERM BP-6173) slant-cultivated by a conventional method was inoculated in the medium and cultivated with shaking at 35 °C for 3 days. The culture medium (100 ml) was inoculated in a 5-liter fermentation tank charged with 3 liters of a liquid medium containing 40% (W/V) of glucose, 1.5% of yeast extracts (manufactured by Asahi Beer Co., Ltd.), and 500 ppm of a antifoam agent CA330 (manufactured by Nippon Yushi Co., Ltd.) and incubated under conditions of 35 °C, air flow rate of 0.5 vvm, and rotation number of 700 rpm, for 4 days. As a result of measurement of erythritol concentration in the culture medium by high performance liquid chromatography, it was found that 175.1 g/L of erythritol was accumulated. During the incubation, no foaming was observed.

Example 21

A liquid medium (100 ml) containing 30% (W/V) of glucose and 1.0% of yeast extracts (manufactured by Asahi Beer Co., Ltd.) charged in a 500 ml Erlenmeyer flask with a cotton plug was sterilized at 120 °C for 20 minutes. A loopful of MCI3440 strain (FERM BP-6175) slant-cultivated by a conventional method was inoculated in the medium and cultivated with shaking at 35°C for 3 days. The culture medium (100 ml) was inoculated in a 5-liter fermentation tank charged with 3 liters of a liquid medium containing 40% (W/V) of glucose, 1.5% of yeast extracts (manufactured by Asahi Beer Co., Ltd.), and 500 ppm of a antifoam agent CA330 (manufactured by Nippon Yushi Co., Ltd.) and incubated under conditions of 35 °C, air flow rate of 0.5 vvm, and rotation number of 700 rpm, for 4 days. As a result of measurement of erythritol concentration in the culture medium by high performance liquid chromatography, it was found that 140.1 g/L of erythritol was accumulated. During the incubation, no foaming was observed.

Example 22

A liquid medium (100 ml) containing 30% (W/V) of glucose and 1.0% of yeast extracts (manufactured by Asahi Beer Co., Ltd.) charged in a 500 ml Erlenmeyer flask with a cotton plug was sterilized at 120 °C for 20 minutes. A loopful of MCI3437 strain (FERM BP-6174) slant-cultivated by a conventional method was inoculated in the medium and cultivated with shaking at 35 °C for 3 days. The culture medium (100 ml) was inoculated in a 5-liter fermentation tank charged with 3 liters of a liquid medium containing 40% (W/V) of glucose, 1.5% of yeast extracts (manufactured by Asahi Beer Co., Ltd.), and 500 ppm of a antifoam agent CA330 (manufactured by Nippon Yushi Co., Ltd.) and incubated under conditions of 35°C, air flow rate of 0.5 vvm, and rotation number of 700 rpm, for 4 days. As a result of measurement of erythritol concentration in the culture medium by high performance liquid chromatography, it was found that 152.1 g/L of erythritol was accumulated. During the incubation, no foaming was observed.

Comparative Example 16

A liquid medium (100 ml) containing 30% (W/V) of glucose and 1.0% of yeast extracts (manufactured by Asahi Beer Co., Ltd.) charged in a 500 ml Erlenmeyer flask with a cotton plug was sterilized at 120 °C for 20 minutes. A loopful of CBS461.67 strain slant-cultivated by a conventional method was inoculated in the medium and cultivated with shaking at 35 °C for 3 days. The culture medium (100 ml) was inoculated in a 5-liter fermentation tank charged with 3 liters of a liquid medium containing 40% (W/V) of glucose, 1.5% of yeast extracts (manufactured by Asahi Beer Co., Ltd.), and 500 ppm of a antifoam agent CA330 (manufactured by Nippon Yushi Co., Ltd.) and incubated under conditions of 35 °C, air flow rate of 0.5 vvm, and rotation number of 700 rpm, for 4 days. Two days after the initiation of the cultivation, serious foaming was observed, and addition of a antifoam agent was unsuccessful in preventing the foaming.

Industrial Applicability

According to the production method of the present invention, erythritol-producing microorganisms which do not form substantial foams during aerobic cultivation can be produced efficiently. Further, the present invention can solve the serious foaming during production of erythritol and allows high yield and inexpensive production of erythritol from raw materials which can be supplied at low costs and without difficulty, such as glucose.

**Claims**

1. A method of producing a microorganism which does not form substantial foams during aerobic cultivation and which has an ability of producing erythritol, comprising the steps of:

   cultivating a microorganism having an ability of producing erythritol in a liquid medium;
   removing a microbial aggregate from the culture; and
   collecting a microorganism which does not form substantial foams during aerobic cultivation from the microorganism remaining in said culture.

2. The method as claimed in claim 1, wherein said step of cultivating said microorganism in the liquid medium, and removing the microbial aggregate from the culture is repeated.

3. The method as claimed in claim 1 or 2, wherein said microorganism is subjected to a mutational treatment prior to the cultivation in the liquid medium.

4. The method as claimed in any one of claims 1 to 3, wherein said microorganism cultivated in the liquid medium is a yeast-like filamentous fungus.

5. The method as claimed in claim 4, wherein said yeast-like filamentous fungus is a microorganism belonging to the genus Moniliella.

6. The method as claimed in claim 5, wherein said microorganism belonging to the genus Moniliella is a microorganism selected from the group consisting of Moniliella pollinis and Moniliella suaveolens var. nigra.

7. The method as claimed in claim 5, wherein said microorganism belonging to the genus Moniliella is a microorganism selected from the group consisting of Moniliella pollinis CBS461.67, Moniliella pollinis MCI3554, Moniliella suaveolens var. nigra CBS223.32, Moniliella suaveolens var. nigra CBS382.36, and Moniliella suaveolens var. nigra CBS223.79.

8. The method as claimed in claim 4, wherein said yeast-like filamentous fungus is a microorganism belonging to the genus Trichosporonoides.

9. The method as claimed in claim 8, wherein said microorganism belonging to the genus Trichosporonoides is a microorganism selected from the group consisting of Trichosporonoides oedocephalis, Trichosporonoides megachiliensis, Trichosporonoides madida, Trichosporonoides nigrescens, and Trichosporonoides spathulata.

10. The method as claimed in claim 8, wherein said microorganism belonging to the genus Trichosporonoides is a microorganism selected from the group consisting of Trichosporonoides oedocephalis CBS649.66, Trichosporonoides oedocephalis CBS568.85, Trichosporonoides megachiliensis CBS567.85, Trichosporonoides megachiliensis ATCC76718, Trichosporonoides madida CBS240.79, Trichosporonoides nigrescens CBS268.81, Trichosporonoides nigrescens CBS269.81, Trichosporonoides spathulata CBS241.79, Trichosporonoides spathulata CBS242.79A, and Trichosporonoides spathulata CBS242.79B.

11. A method of producing an erythritol-producing microorganism, comprising the steps of:

collecting in a liquid medium a microorganism having an ability of producing erythritol and physical properties such that when fractionated with water and a water-insoluble solvent, said microorganism remains in a water layer in an amount of at least 20% and/or a microorganism which has a hydrophobicity of 80% or less; and collecting from said microorganism(s) a microorganism which does not form substantial foams during aerobic cultivation.

12. The method as claimed in claim 11, further comprising the steps of:

cultivating a microorganism having an ability of producing erythritol in a liquid medium; and removing a microbial aggregate from said culture.

13. The method as claimed in claim 12, wherein said step of cultivating the microorganism in the liquid medium, and removing the microbial aggregate from the culture is repeated.

14. The method as claimed in claim 12 or 13, wherein said microorganism is subjected to a mutational treatment prior to the cultivation in the liquid medium.

15. The method as claimed in any one of claims 11 to 14, wherein said microorganism cultivated in the liquid medium is a yeast-like filamentous fungus.

16. The method as claimed in claim 15, wherein said yeast-like filamentous fungus is a microorganism belonging to the genus Moniliella.

17. The method as claimed in claim 16, wherein said microorganism belonging to the genus Moniliella is a microorganism selected from the group consisting of Moniliella pollinis and Moniliella suaveolens var. nigra.

18. The method as claimed in claim 16, wherein said microorganism belonging to the genus Moniliella is a microorganism selected from the group consisting of Moniliella pollinis CBS461.67, Moniliella pollinis MCI3554, Moniliella suaveolens var. nigra CBS223.32, Moniliella suaveolens var. nigra CBS382.36, and Moniliella suaveolens var. nigra CBS223.79.

19. The method as claimed in claim 15, wherein said yeast-like filamentous fungus is a microorganism belonging to the genus Trichosporonoides.

20. The method as claimed in claim 19, wherein said microorganism belonging to the genus Trichosporonoides is a microorganism selected from the group consisting of Trichosporonoides oedocephalis, Trichosporonoides megachiliensis, Trichosporonoides madida, Trichosporonoides nigrescens, and Trichosporonoides spathulata.

21. The method as claimed in claim 19, wherein said microorganism belonging to the genus Trichosporonoides is a microorganism selected from the group consisting of Trichosporonoides oedocephalis CBS649.66, Trichosporonoides oedocephalis CBS568.85, Trichosporonoides megachiliensis CBS567.85, Trichosporonoides megachiliensis ATCC76718, Trichosporonoides madida CBS240.79, Trichosporonoides nigrescens CBS268.81, Trichosporonoides nigrescens CBS269.81, Trichosporonoides spathulata CBS241.79, Trichosporonoides spathulata CBS242.79A, and Trichosporonoides spathulata CBS242.79B.

22. An erythritol-producing microorganism obtained by a method as claimed in any one of claims 1 to 10, wherein microorganism does not form substantial foams during aerobic cultivation.

23. An erythritol-producing microorganism which does not form substantial foams during aerobic cultivation, said microorganism being obtained by a method as claimed in claim 7 and selected from the group consisting of MCI3371 (FERM BP-6173) which is a mutant of Moniliella pollinis CBS461.67, MCI3555 (FERM BP-6171) which is a mutant of Moniliella pollinis MCI3554, MCI3598 which is a mutant of Moniliella suaveolens var. nigra CBS223.32, MCI3599 which is a mutant of <u>Moniliella</u> <u>suaveolens</u> <u>var</u>. <u>nigra</u> CBS382.36, and MCI3600 which is a mutant of <u>Moniliella</u> <u>suaveolens</u> <u>var</u>. <u>nigra</u> CBS223.79.

24. An erythritol-producing microorganism which does not form substantial foams during aerobic cultivation, said microorganism being obtained by a method as claimed in claim 10 and selected from the group consisting of MCI3439 (FERM BP-6308), which is a mutant of <u>Trichosporonoides</u> <u>oedocephalis</u> CBS649.66, MCI3440 (FERM BP-6175), which is a mutant of <u>Trichosporonoides</u> <u>oedocephalis</u> CBS568.85, MCI3369 (FERM BP-6172), which is a mutant of <u>Trichosporonoides</u> <u>megachiliensis</u> CBS567.85, MCI3604, which is a mutant of <u>Trichosporonoides</u> <u>megachiliensis</u> ATCC76718, MCI3441 (FERM BP-6309), which is a mutant of <u>Trichosporonoides</u> <u>madida</u> CBS240.79, MCI3437 (FERM BP-6174), which is a mutant of <u>Trichosporonoides</u> <u>nigrescens</u> CBS268.81, MCI3438 (FERM BP-6307), which is a mutant of <u>Trichosporonoides</u> <u>nigrescens</u> CBS269.81, MCI3601, which is a mutant of <u>Trichosporonoides</u> <u>spathulata</u> CBS241.79, MCI3602, which is a mutant of <u>Trichosporonoides</u> <u>spathulata</u> CBS242.79A, and MCI3603, which is a mutant of <u>Trichosporonoides</u> <u>spathulata</u> CBS242.79B.

25. An erythritol-producing microorganism which does not form substantial foams during aerobic cultivation, said microorganism being obtained by a method as claimed in any one of claims 11 to 21.

26. A method of producing erythritol, comprising the steps of:

cultivating an erythritol-producing microorganism as claimed in any one of claims 22 to 26 or a mutant thereof in a medium; and
collecting erythritol from said culture.

27. An erythritol-producing microorganism belonging to the genus <u>Moniliella</u>, having an ability of producing erythritol, wherein microorganism does not form substantial foams during aerobic cultivation.

28. An erythritol-producing microorganism belonging to the genus <u>Moniliella</u> having an ability of producing erythritol, wherein microorganism has physical properties such that when fractionated with water and a water-insoluble solvent, said microorganism remains in a water layer in an amount of at least 20% and/or a hydrophobicity of 80% or less, and which microorganism does not form substantial foams during aerobic cultivation.

29. An erythritol-producing microorganism which is a mutant of a microorganism selected from the group consisting of <u>Moniliella</u> <u>pollinis</u> and <u>Moniliella</u> <u>suaveolens</u> <u>var</u>. <u>nigra</u>, said mutant having an ability of producing erythritol and forming no substantial foam during aerobic cultivation.

30. An erythritol-producing microorganism which is a mutant of a microorganism selected from the group consisting of <u>Moniliella</u> <u>pollinis</u> CBS461.67, <u>Moniliella</u> <u>pollinis</u> MCI3554, <u>Moniliella</u> <u>suaveolens</u> <u>var</u>. <u>nigra</u> CBS223.32, <u>Moniliella</u> <u>suaveolens</u> <u>var</u>. <u>nigra</u> CBS382.36, and <u>Moniliella</u> <u>suaveolens</u> <u>var</u>. <u>nigra</u> CBS223.79, said mutant having an ability of producing erythritol and forming no substantial foam during aerobic cultivation.

31. An erythritol-producing microorganism which is selected from the group consisting of MCI3371 (FERM BP-6173), which is a mutant of <u>Moniliella</u> <u>pollinis</u> CBS461.67, MCI3555 (FERM BP-6171), which is a mutant of <u>Moniliella</u> <u>pollinis</u> MCI3554, MCI3598, which is a mutant of <u>Moniliella</u> <u>suaveolens</u> <u>var</u>. <u>nigra</u> CBS223.32, MCI3599, which is a mutant of <u>Moniliella</u> <u>suaveolens</u> <u>var</u>. <u>nigra</u> CBS382.36, and MCI3600, which is a mutant of <u>Moniliella</u> <u>suaveolens</u> <u>var</u>. <u>nigra</u> CBS223.79, said mutant forming no substantial foam during aerobic cultivation.

## INTERNATIONAL SEARCH REPORT

| | International application No. |
|---|---|
| | PCT/JP98/01534 |

A. CLASSIFICATION OF SUBJECT MATTER
Int.Cl⁶ C12N1/16 // C07C31/24

According to International Patent Classification (IPC) or to both national classification and IPC

B. FIELDS SEARCHED

Minimum documentation searched (classification system followed by classification symbols)
Int.Cl⁶ C12N1/16, C07C31/24

Documentation searched other than minimum documentation to the extent that such documents are included in the fields searched

Electronic data base consulted during the international search (name of data base and, where practicable, search terms used)
BIOSIS (DIALOG), WPI (DIALOG)

C. DOCUMENTS CONSIDERED TO BE RELEVANT

| Category* | Citation of document, with indication, where appropriate, of the relevant passages | Relevant to claim No. |
|---|---|---|
| Y | JP, 63-196298, A (Director General of National Food Research Institute, Ministry of Agriculture, Forestry and Fisheries and another), August 15, 1988 (15. 08. 88) & EP, 262463, A1 & JP, 63-68071, A & AU, 8777768, A & DK, 8704679, A & US, 4939091, A & JP, 3-43091, A & US, 5036011, A & KR, 9007936, B & DK, 168490, B & JP, 7-203978, A | 1-31 |
| Y | JP, 6-141850, A (Hitachi Ltd.), May 24, 1994 (24. 05. 94) (Family: none) | 1-26 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| June 1, 1998 (01. 06. 98) | June 9, 1998 (09. 06. 98) |

| Name and mailing address of the ISA/ | Authorized officer |
|---|---|
| Japanese Patent Office | |
| Facsimile No. | Telephone No. |

Form PCT/ISA/210 (second sheet) (July 1992)

21

## INTERNATIONAL SEARCH REPORT

| International application No. |
| --- |
| PCT/JP98/01534 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP, 60-110298, A  (CPC International Inc.), June 15, 1985 (15. 06. 85) & EP, 136804, A1  &  DK, 8404027, A & FI, 8403313, A  &  BR, 8404195, A & ES, 8600397, A | 5-7, 16-18, 22-23, 25-31 |
| Y | AOKI, M.A.Y. et al., "Microbial transformation of sucrose and glucose to erythritol", BIOTECHNOLOGY LETTERS (1993) Vol. 15, No. 4 p.383-388 | 8-10, 19-22, 24-26 |

Form PCT/ISA/210 (continuation of second sheet) (July 1992)